(19) Europäisches Patentamt
European Patent Office
Office européen des brevets

(11) Veröffentlichungsnummer : **0 075 170**
**B1**

(12) # EUROPÄISCHE PATENTSCHRIFT

(45) Veröffentlichungstag der Patentschrift : **26.03.86**

(51) Int. Cl.[4] : **C 07 D233/16**, C 07 D233/06

(21) Anmeldenummer : **82108187.4**

(22) Anmeldetag : **06.09.82**

(54) **Imidazolinderivate.**

(30) Priorität : **17.09.81 DE 3137044**

(43) Veröffentlichungstag der Anmeldung : **30.03.83 Patentblatt 83/13**

(45) Bekanntmachung des Hinweises auf die Patenterteilung : **26.03.86 Patentblatt 86/13**

(84) Benannte Vertragsstaaten : **CH DE FR GB LI**

(56) Entgegenhaltungen :
**DE-A- 1 619 078**
**FR-A- 1 501 151**
**US-A- 4 267 350**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Boehmke, Günther, Dr.**
**Kurt-Schumacher-Ring 152**
**D-5090 Leverkusen 1 (DE)**
Erfinder : **Bode, Klaus-Dieter, Dr.**
**Droste-Hülshoff-Strasse 9**
**D-5068 Odenthal (DE)**
Erfinder : **Kortmann, Wilfried**
**Auf der Driesche 3**
**D-5800 Hagen 5 (DE)**

**EP 0 075 170 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schrifflich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Imidazolinderivate der Formel (I)

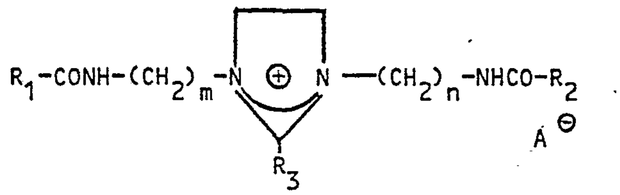

(I)

in der

$R_1$, $R_2$, $R_3$ $C_{15}$-$C_{19}$-Alkyl,
$R_3$ zusätzlich Methyl,
m, n 2 oder 3 und
$A^{\ominus}$ ein Anion bezeichnen.

Geeignete Reste $R_1$, $R_2$, $R_3$ sind beispielsweise $CH_3(CH_2)_{16}$ und $CH_3(CH_2)_{14}$. $R_3$ steht insbesondere für $CH_3$.

Geeignete Anionen sind insbesondere Formiat, Acetat, Phosphat, Phosphit, Phosphonat, Sulfonat, Toluolsulfonat oder Benzoat.

Die Herstellung der neuen Verbindungen kann z. B. in der Weise erfolgen, daß man Verbindungen der Formel (II)

$$R_1{-}CONH{-}\ (CH_2)_m{-}NH{-}CH_2{-}CH_2{-}NH{-}(CH_2)_n{-}NHCO{-}R_2 \qquad (II)$$

in der $R_1$, $R_2$, m und n die zu Formel (I) angegebenen Bedeutungen haben, mit Acylierungsmitteln der Formel (III)

$$R_3COX \qquad (III)$$

in der $R_3$ die zu Formel (I) angegebene Bedeutung hat und X OH, Cl oder Br ist, zu Verbindungen der Formel IV

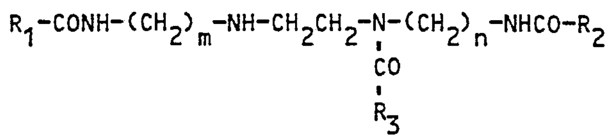

(IV)

in der $R_1$, $R_2$, $R_3$, m und n die zu Formel I angegebenen Bedeutungen besitzen, acyliert, und man anschließend mit Säuren HA, in der A die zu Formel (I) angegebene Bedeutung besitzt, den Imidazolinring schließt.

Die neuen Verbindungen finden Verwendung als Weichmacher für textile Gewirke, Gewebe oder Vliese aus natürlichen und synthetischen Fasern sowie für Papier und Leder. Bevorzugt eingesetzt werden sie in Form leicht wasserlöslicher Schuppen sowie in wässriger, organischer oder wässrigorganischer, insbesondere konzentrierter organischer Lösung, wobei die Lösung weitere Emulgatoren, Hydrophobierungsmittel und/oder optische Aufheller enthalten kann. Erwähnenswert ist weiterhin die Verwendung der neuen Verbindungen als Bestandteile von pulverförmigen oder flüssigen Waschmitteln, die gegebenenfalls einen optischen Aufheller enthalten.

Durch die Kettenlängen der Reste $R_1$, $R_2$ und $R_3$ in Formel (I) können viele Eigenschaften der Weichmacher in der gewünschten Richtung beeinflußt werden, wie eine leichte Löslichkeit, eine große Hydrophile in der ausgerüsteten Ware, eine bessere Applikation aus kurzer Flotte, ein besseres Ausziehverhalten aus langer Flotte, einen sehr weichen Griff der ausgerüsteten Ware, eine sehr geringe Härteempfindlichkeit, eine geringe pH-Empfindlichkeit, eine gute Sprödigkeit zur Verschuppung des Produktes und eine leichte Auflösung der Schuppenware.

Ein weiterer Vorteil der erfindungsgemäßen Weichmacher ist die geringe Beeinflussung des Weißgrades einer aufgehellten Ware bzw. im Waschprozeß wieder aufgehellten und wieder weichgemachten Ware. Deshalb lassen sich mit diesen Weichmachern auch leicht Lösungen herstellen, die optische Aufheller enthalten, ohne daß der Weißgrad bei der Textilbehandlung wesentlich beeinflußt wird.

Die Verwendung bestimmter Imidazolinium-Verbindungen als Weichmacher ist bereits bekannt. In der US-A-4 267 350 werden Amide von durch Imidazolinium-Reste substituierten Alkylcarbonsäuren und in der DE-A-16 19 078 Mono-N-acyl-aminoalkyl-imidazolinium-Salze zu diesem Zweck eingesetzt.

## Beispiel 1

540 g Stearinsäure (2 Mol eines technischen Talgfettsäuregemisches, SZ = 207) und 146 g Triethylentetramin (1 Mol) werden unter Stickstoffatmosphäre auf etwa 175 °C erhitzt. Es spalten sich 36 g Wasser ab, die ohne Vakuum abdestilliert werden. Eine IR-Analyse weist auf die Bildung des 1, ω-Acylderivates, ohne bei 6,4 μ eine Imidazolin-Bande anzuzeigen. Dazu läßt man 120 g Essigsäure einlaufen und destilliert weiteres Wasser bei 175-185 °C ab. Nach etwa 2 Stunden wird das Destillat auf Essigsäure untersucht. Die festgestellte Menge wird in die Reaktionsmischung nachgeführt und die Reaktion zu Ende geführt. Das leicht erstarrende Endprodukt kann auf eine Kühlwalze zu Schuppen verarbeitet werden. Diese lösen sich leicht in kaltem Wasser zu einer 1 : 10 verdünnten Lösung, wenn man die Schuppen mit kaltem Wasser übergießt und mehrere Stunden stehen läßt und dann kurze Zeit homogen rührt.

Der Nachweis für den Ringschluß gelingt im IR-Spektrum nicht, da die charakteristische Bande durch die Säureamidbanden überdeckt wird. Dagegen ist im UV-Spektrum das Auftreten eines neuen Maximums bzw. einer stark ausgeprägten Schulter bei 230-232 nm ein sicherer Nachweis für den 1,2,3-substituierten Imidazolinring.

## Beispiel 2

10 g des Produktes aus Beispiel 1 werden in 90 g Wasser von 17 °dH und 18-20 °C eingestreut. Nach 15 Minuten Rührzeit sind die Schuppen in der Voltage gut verteilt und angenetzt. Das Rührwerk wird abgestellt. Nach 8 h Quell- und Lösevorgang wird für 10 Minuten das Rührwerk angestellt und die Emulsion homogenisiert.

Die resultierende Emulsion zeigt keine Ausfällungen und/oder ungelösten Bestandteile und ist für die Verwendung als Weichmachungsmittel für Textilien einsetzbar.

## Beispiel 3

Auf einem Labordüsenfärbeapparat werden 200 g Co-Wirkware nach bekannten Verfahren mit Reaktivfarbstoffe gefärbt, anschließend geseift und mehrfach heiß und kalt gespült und in der letzten Spülflotte belassen.

Dann werden 4 g der 10 %igen Emulsion nach Beispiel 2 in 100 ccm 35 °C warmes Wasser eingegossen und durch leichtes Rühren verteilt.

Die 4 g Emulsion entsprechen 2 % Weichmachungsmittel bezogen auf das Warengewicht.

Diese Lösung wird in das Ansatzgefäß gegossen und in den Düsenfarbapparat eingespült. Während einer Behandlungszeit von 20 Minuten bei 35 °C entstehen nur minimale Schaummengen, die den Warenlauf nicht beeinträchtigen. Ausfällungen, z. B. Brechen der Emulsion durch die hohe Scherkraftwirkung der Pumpe werden nicht beobachtet. Nach Beedigung der Behandlungszeit wird die Flotte abgelassen, die Ware entnommen und in einer Haushaltsschleuder auf 25 % RF entwässert. Das Textil wird in einem Trockenschrank bei 120 °C mit durch Ventilatoren bewegter Luft getrocknet.

Nach einer Klimatisierung von 24 Stunden bei 65 % Restfeuchte wird das Stoffmuster bewertet.

A) Griff : Angenehmer weicher, voluminöser Griff

B) Hydrophilie : 0,5 cm destilliertes Wasser werden mittels einer Pipette auf das Gewebe aufgetropft und die Einsinkzeit bestimmt : Herkömmliches kationisches Weichmachungsmittel auf Basis Distearyldimethylammoniumchlroid : Einsinkzeit 60-180 Sekunden ; erfindungsgemäßes Produkt : Einsinkzeit 25-35 Sekunden.

## Beispiel 4

Co-Frottierware wird auf einer Haspelkufe mit einem handelsüblichen anionischen Weißtöner auf Basis Stilbendisulfonsäure behandelt. Danach wird kurz gespült und 1,5 % vom Warengewicht der 10 %igen Emulsion nach Beispiel 2, die in 100 ccm 35 °C warmes Wasser eingegossen und durch leichtes Rühren verteilt werden, dem Spülbad zugegeben. Nach Abschluß der Behandlung wird geschleudert, bei 120 °C getrocknet und klimatisiert.

Im Gegensatz zur Behandlung mit einem handelsüblichen Weichmachungsmittel auf Basis Distearyldimethylammoniumchlorid (Farbtonumschlag nach Gelb) erfolgt bei der vorstehend beschriebenen Behandlung mit dem erfindungsgemäßen Weichmacher eine deutlich geringere Beeinflussung des Weißgrades (siehe auch Tabelle 1).

## Beispiel 5

Polyacrylnitril-Hochbausch-Garn wird auf einem Garnfärbeapparat gefärbt und gespült. 3 % (bezogen auf das Garngewicht) der gemäß Beispiel 2 erhaltenen Zubereitung werden mit Wasser verdünnt und dem Spülbad zugefügt. Nacht 20 Minuten und einer Behandlungstemperatur von 35 °C wird geschleudert und bei 120 °C getrocknet. Nach der Klimatisierung von 24 h bei 65 % Restfeuchte wurde der Oberflächenwiderstand (ROT) nach DIN 54 345, Blatt 1 gemessen: $2{,}5 \times 10^8$ Ohm. Damit ist eine ausreichende Antielektrostatik sowohl für die Weiterverarbeitung (z. B. Weben und Stricken) wie auch für den Tragekomfort gegeben.

Beispiel 6

Wie die nachfolgende Tabelle 1 zeigt, wird bei der Nachbehandlung einer mit einem handelsüblichen pulverförmigen Waschmittels gewaschenen und aufgehellten Wäsche mit einem handelsüblichen Weichmachungsmittel auf Basis Distearyldimethylammoniumchlorid der Aufhellungsgrad deutlich vermindert. Selbst die Zugabe eines handelsüblichen Weichmachers auf Basis Stilbendisulfonsäure behebt diesen Effekt nicht vollständig. Bei Nachbehandlung der Wäsche mit dem gemäß Beispiel 1 erhaltenen erfindungsgemäßen Weichmacher wird der Aufhellungseffekt kaum beeinträchtigt; bei Zugabe eines handelsüblichen Aufhellers tritt sogar ein weiterer Aufbau des Aufhellungseffektes ein.

(Siehe Tabelle 1 Seite 5 f.)

Tabelle 1

Weißgrad (W 6') auf gebleichtem Baumwollgewebe (W 6' = 41)

| Wasch-zyklen | gewaschen mit 5g/l A | nachbehandelt mit 2 g/l B | nachbehandelt mit 2 g/l B+0,5 % C | nachbehandelt mit 2 g/l D | nachbehandelt mit 2 g D + 0,5 % C |
|---|---|---|---|---|---|
| 1 x | 133 | 93 | 96 | 123 | 143 |
| 2 x | 167 | 129 | 148 | 165 | 175 |

A = handelsübliches pulverförmiges Waschmittel enthaltend optische Aufheller (Omo(R))
B = handelsübliche Weichmachungsmittel auf Basis Distearyldimethylammoniumchlorid
C = anionischer Weißtöner auf Basis Stilbendisulfonsäure
D = erfindungsgemäßes Weichmachungsmittel gemäß Beispiel 1

Beispiel 7

Wenn der erfindungsgemäße Weichmacher aus Beispiel 1 einem flüssigen Waschmittel zugesetzt wird, wird im Gegensatz zu einem handelsüblichen Weichmacher noch ein Aufbau des Weißgrades beobachtet (s. Tabelle 2).

Tabelle 2

Weißgrad (WG 6') auf gebleichtem Baumwollgewebe (WG' = 41)

| Wasch-zyklen | gewaschen mit E | gewaschen mit E enthaltend 7,5 % B | gewaschen mit E enthaltend 7,5 % D |
|---|---|---|---|
| 1 x | 149 | 144 | 149 |
| 3 x | 167 | 164 | 173 |

E = flüssiges Waschmittel enthaltend 25 % nichtionisches Tensid, 4 % Triethanolamin, 11 % Ethanol, 15 % anionisches Tensid und 0,5 % Weißtöner auf Basis Stilbendisulfonsäure
B = handelsübliches Waschmittel auf Basis Distearyldimethylammoniumchlorid
D = erfindungsgemäßes Weichmachungsmittel gemäß Beispiel 1

Beispiel 8

621 g Stearinsäure (2,3 Mol eines technischen Talgfettsäuregemisches, SZ = 207) und 146 g Triethylentetramin (1 Mol) wurden nach den Bedingungen des Beispiels 1 zur Reaktion gebracht. Als Essigsäure wurden 102 g ( = 1,7 Mol) eingesetzt. Die Cyclisierung wird wie unter den Bedingungen des Beispiel 1 durchgeführt. Bei diesem Produkt ist $R_3$ in 0,7 Mol $CH_3$ und 0,3 Mol $C_{17}H_{35}$-Gruppen aufgeteilt. Vor dem Erkalten werden in dem Produkt 24 g phosphorige Säure gelöst und dann die Verschuppung auf einer Kühlwalze angeschlossen. Man erhält einen spröden, hellen und sich leicht auflösenden Weichmacher (6.4µ (IR)).

Nach Ausrüstung eines Polyamidmonofils wird eine Haft-Reibung von 0,07 gemessen, während Distearyldimethylammoniumchlorid unter den gleichen Bedingungen einen Wert von 0,14 anbringt (Faden-Faden-Reibung).

## Patentansprüche

1. Imidazolinderivate der Formel (I)

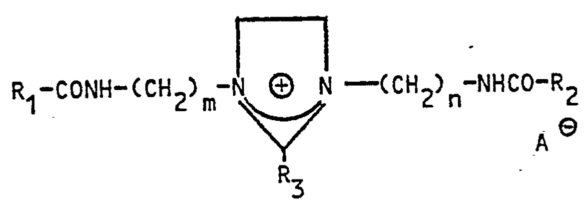

(I)

in der
$R_1$, $R_2$, $R_3$ $C_{15}$-$C_{19}$-Alkyl,
$R_3$ zusätzlich Methyl,
m, n 2 oder 3 und
A⊖ ein Anion bezeichnen.

2. Verfahren zur Herstellung von Verbindungen gemäß Anspruch 1, dadurch gekennzeichnet, daß man Verbindungen der Formel (II)

$$R_1—CONH—(CH_2)_m—NH—CH_2CH_2—NH—(CH_2)_n—NHCO—R_2 \quad (II)$$

in der $R_1$, $R_2$, m und n die zu Formel (I) angegebenen Bedeutungen haben, mit Acylierungsmitteln der Formel (III)

$$R_3COX \qquad (III)$$

in der $R_3$ die zu Formel (I) angegebene Bedeutung hat und X OH, Cl oder Br ist, zu Verbindungen der Formel (IV)

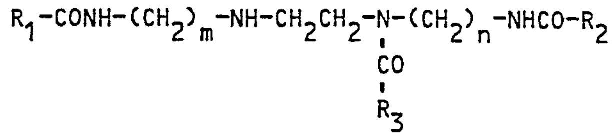

(IV)

in der $R_1$, $R_2$, $R_3$, m und n die zu Formel (I) angegebenen Bedeutungen besitzen, acyliert, und man anschließend mit Säuren HA, in der A die zur Formel (I) angegebene Bedeutung hat, den Imidazolinring schließt.

3. Verwendung der Verbindungen gemäß Anspruch 1 als Weichmacher für textile Gewirke, Gewebe oder Vliese.

4. Verwendung der Verbindungen gemäß Anspruch 1 als Weichmacher für Papier.

5. Verwendung der Verbindungen gemäß Anspruch 1 als Weichmacher für Leder.

6. Verwendung der Verbindungen gemäß Anspruch 3 in einer Flüssigformulierung mit optischen Aufhellern.

7. Verwendung der Verbindungen gemäß Anspruch 3 als Bestandteil eines flüssigen Waschmittels, das optische Aufheller enthalten kann.

**Claims**

1. Imidazoline derivatives of the formula (I)

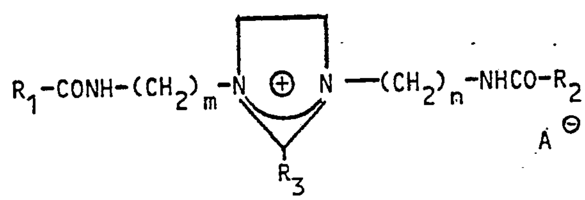

(I)

in which
$R_1$, $R_2$ and $R_3$ designate $C_{15}$-$C_{19}$-alkyl,
$R_3$ additionally designates methyl,
m and n designate 2 or 3 and
A⊖ designates an anion.

2. Process for the preparation of compounds according to Claim 1, characterised in that compounds of the formula (II)

$$R_1—CONH—(CH_2)_m—NH—CH_2CH_2—NH—(CH_2)_n—NHCO—R_2 \qquad (II)$$

in which $R_1$, $R_2$, m and n have the meanings given for formula (I), are acylated with acylating agents of the formula (III)

$$R_3COX \qquad (III)$$

in which $R_3$ has the meaning given for formula (I) and X is OH, Cl or Br, to give compounds of the formula (IV)

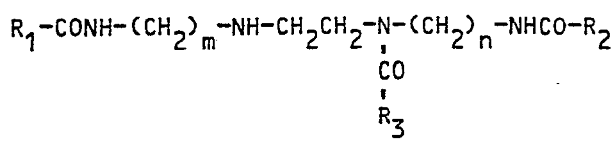

(IV)

in which $R_1$, $R_2$, $R_3$, m and n have the meanings given for formula (I), and the imidazoline ring is then closed using acids HA, in which A has the meaning given for formula (I).

3. Use of the compounds according to Claim 1 as softeners for knitted textiles, woven textiles or nonwoven textiles.

4. Use of the compounds according to Claim 1 as softeners for paper.

5. Use of the compounds according to Claim 1 as softeners for leather.

6. Use of the compounds according to Claim 3 in a liquid formulation containing optical brighteners.

7. Use of the compounds according to Claim 3 as constituents of a liquid detergent which can contain optical brighteners.

**Revendications**

1. Dérivés d'imidazoline de formule (I)

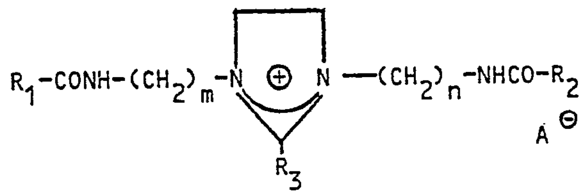

(I)

dans laquelle

$R_1$, $R_2$ et $R_3$ représentent des groupes alkyles en $C_{15}$-$C_{19}$

$R_3$ représente en outre un groupe méthyle

m et n sont chacun égal à 2 ou 3 et

$A^\ominus$ représente un anion.

2. Procédé pour la fabrication de composés selon la revendication 1, caractérisé en ce que l'on acyle des composés de formule (II)

$$R_1—CONH—(CH_2)_m—NH—CH_2—CH_2—NH—(CH_2)_n—NHCO—R_2 \quad (II)$$

dans laquelle $R_1$, $R_2$, m et n ont les significations indiquées pour la formule (I), avec des agents acylants de formule (III)

$$R_3COX \quad (III)$$

dans laquelle $R_3$ a la signification indiquée pour la formule (I) et X représente OH, Cl ou Br, en composés de formule (IV)

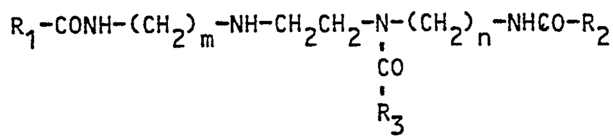

(IV)

dans laquelle $R_1$, $R_2$, $R_3$, m et n ont les significations indiquées pour la formule (I) et on ferme ensuite le cycle imidazoline avec des acides HA, dans lesquels A a la signification indiquée pour la formule (I).

3. Utilisation des composés selon la revendication 1 comme assouplissants pour les tricots, tissus ou nappes textiles.

4. Utilisation des composés selon la revendication 1 comme assouplissants pour le papier.

5. Utilisation des composés selon la revendication 1 comme assouplissants pour le cuir.

6. Utilisation selon la revendication 3 dans une composition liquide avec des azureurs optiques.

7. Utilisation selon la revendication 3 comme composant d'un produit de lavage liquide qui peut contenir des azureurs optiques.